Europäisches Patentamt

(19) European Patent Office

Office européen des brevets

(11) Publication number: **0 071 654**
**B1**

(12) **EUROPEAN PATENT SPECIFICATION**

(45) Date of publication of patent specification: **02.07.86**

(21) Application number: **81106089.6**

(22) Date of filing: **04.08.81**

(51) Int. Cl.⁴: **G 01 N 33/53, G 01 N 33/531**

54 **Methods and materials for detection of multiple sclerosis.**

43 Date of publication of application:
**16.02.83 Bulletin 83/07**

(45) Publication of the grant of the patent:
**02.07.86 Bulletin 86/27**

84 Designated Contracting States:
**DE FR GB**

58 References cited:
**WO-A-80/02457**
**US-A-4 136 160**
**US-A-4 294 818**

**CHEMICAL ABSTRACTS, vol. 88, no. 7, 13th February 1978, abstract 48671p, page 372, COLUMBUS OHIO (US), E. FRICK et al.: "The pathogenesis of multiple sclerosis. Antibody dependent cytotoxicity of lymphocytes towards the basic myelin protein in multiple sclerosis"**

**CHEMICAL ABSTRACTS, vol. 90, no. 15, 9th April 1979, abstract 119661q, page 489, COLUMBUS OHIO (US), G.C. EBERS et al.: "Oligoclonal immunoglobulins in subacute sclerosing panencephalitis and multiple sclerosis: a study of idiotypic determinants"**

73 Proprietor: **McMichael, John**
**Post Office Box 81**
**Cambridge Springs Pennsylvania 16403 (US)**
73 Proprietor: **Kline, Ellis L.**
**203 Elm Street**
**Pendleton, South Carolina 29670 (US)**
73 Proprietor: **Spaulding, James G.**
**3522 Janice Drive**
**Ruston, Louisiana 71270 (US)**

72 Inventor: **McMichael, John**
**Post Office Box 81**
**Cambridge Springs Pennsylvania 16403 (US)**
Inventor: **Kline, Ellis L.**
**203 Elm Street**
**Pendleton, South Carolina 29670 (US)**
Inventor: **Spaulding, James G.**
**3522 Janice Drive**
**Ruston, Louisiana 71270 (US)**

74 Representative: **Brown, John David et al**
**FORRESTER & BOEHMERT Widenmayerstrasse 4/I**
**D-8000 München 22 (DE)**

Courier Press, Leamington Spa, England.

**Description**

Background

The present invention relates generally to diagnosis of a multiple sclerosis disease state in humans and more specifically to novel antigen and antibody preparations and immunological reagents containing same which are useful in diagnosis of multiple sclerosis.

Multiple sclerosis, sometimes referred to as disseminated sclerosis, is a slowly progressive disease of the central nervous system characterized morphologically by disseminated patches of demyelinization in the brain and spinal cord and clinically by multiple symptoms and signs with remissions and exacerbations.

The etiology of multiple sclerosis is essentially unknown and the disease has been variously attributed to: autoimmune mechanisms; infection by a slow virus; toxic agents such as metallic poisons; metabolic elements such as myelin-splitting factor; and vascular lesions resulting from abnormal blood clotting mechanisms.

Among the varied symptoms of the multiple sclerosis disease state are sensory (especially visual) disorders, spastic weakness of limbs, cerebellar ataxia, nystagmus, bladder dysfunction, mood disorders and combinations of two or more such symptoms.

Diagnosis of the disease state is virtually impossible owing to the overlap of the above-noted symptoms of multiple sclerosis and similar symptoms of other disease states. Diagnosis of the disease state is most frequently premised upon a "classic" history of remissions and exacerbations of the various symptoms over a period of years, combined with systematic elumination of other possible disease involvements which give rise to similar symptoms. Collateral testing at substantial cost and often physical discomfort to the patient is performed in order to "eliminate" e.g., intracranial lesions, cerebrovascular accidents, acoustic neuroma, cerebellar tumors, gliomas of the brain stem, spinal cord tumors, amyotrophic lateral sclerosis, syphillis, pernicious anemia, arthritis of the cervical spine, ruptured intervertebral disk, platybasia, and hereditary ataxia as the source of symptoms.

Substantial efforts have been directed toward development of diagnostic methods and materials useful in the early diagnosis of multiple sclerosis. Positive results in colloidal gold tests on cerebrospinal fluids are considered supportive, but not dispositive of, positive diagnosis. The same is true of testing for elevated gamma globulin in cerebrospinal fluids—the test tends to verify diagnosis in advanced cases but is not helpful in early diagnosis. Similarly, active demyelinization associated with the disease frequently is signified by elevation of analytical results in basic protein assay testing of spinal fluid, but test levels drop rapidly one acute exacerbation is over. Non-dispositive correlations have been made between presence of the disease state and elevated levels of measles antibodies in serum and cerebrospinal fluids. Finally, certain researchers have proposed that electron microscopic examination of lymphocytes for certain distinct morphological changes may provide a fruitful basis for diagnosis of multiple sclerosis.

The character of the most recent advances in the art is exemplified by the disclosures of Angers, et a., reported in Chemical & Engineering News, page 22, April 9, 1979. Briefly summarizes, Angers, et al. assert the usefulness of a leukocyte adherence inhibition (LAI) test using an essentially non-specific blood extract (so-called "Multiple Sclerosis Related Material") from the blood of multiple sclerosis patients in relapsing or progressive disease states. The LAI test involves measurement of decreasing ability of test sample erythrocytes to adhere to a glass surface after incubation in MSRM. The rather complicated, time consuming and expensive procedure is alleged to be approximately ninety percent accurate in identifying multiple sclerosis patients and 95 percent accurate in identifying non-multiple sclerosis patients.

There therefore exists a most substantial need in the art for diagnostic methods and materials for rapidly, simply and accurately determining the presence of a multiple sclerosis disease state. Desirably, such methods should be highly specific for multiple sclerosis (i.e., should not generate false positive results in the instance of other central nervous system diseases). Further, such methods should be capable of ascertaining the presence of the disease state in its early stages, should be operatively independent of disease exacerbation and remission, should not involve painful or hazardous withdrawals of patient tissue samples, and should preferably involve standardized laboratory techniques which do not require expensive or difficult-to-operate apparatus.

Brief summary

According to a principal aspect of the present invention, antibody preparations are provided which are specifically immunologically reactive with antigenic substances associated with lymphocytes of patients having multiple sclerosis wherein antibodies are provided as a component of heterologous species serum. The antibody preparations are employed to develop diagnostic reagents suitable for use in standardized immunological assays applied to lymphocytes obtained from patients suspected of having the disease.

Other aspects and advantages of the present invention will be apparent to those skilled in the art upon consideration of the following detailed description thereof.

Detailed description

The operative basis of the present invention resides at least in part in the discovery of an antigenic substance uniquely associated with the blood (and predominantly the lymphocytes) of human patients clinically diagnosed as suffering from multiple sclerosis. The antigen was further discovered to be capable of prompting quantitative formation of specific antibodies in immunologically active, heterologous species animals (e.g., rabbits). Preparations containing the specific antibodies so generated were found to be immunologically reactive with lymphocytes of multiple sclerosis patients essentially independently of the stage of disease development or the remitted or exacerbated state of the patient at the time of lymphocyte sampling. Significantly, the antibody preparations are essentially non-reactive immunologically with non-multiple sclerosis patients, even those with central nervous system disorders frequently mis-diagnosed relative to multiple sclerosis.

Antibody preparations of the present invention may be suitably employed in a variety of immunological procedures including immunodiffusion assays, fluorescent antibody assays, radioimmunoassays and the like, each of which would involve development of reagents appropriate for such use.

The following examples illustrate practice of the invention according to certain presently preferred procedures. More specifically, they treat: preparation of lymphocytes for use either as an antigenic inoculant or as a patient test sample; development of specific antibody preparations; and use of antibody preparations in an immunodiffusion assay.

Example 1

Lymphocytes employed as antigenic inoculants to develop antibody preparations and lymphocytes employed in diagnostic testing according to the present invention may identically be prepared by the following exemplary procedure. A 10 ml sample of venous blood is collected in an evacuated tube and thoroughly mixed with, e.g., 0.5 ml of 0.1M sodium citrate. Lymphocytes are separated from the blood using Lymphocyte Preparation Medium (Bionetics) and, following low speed centrifugation, the lymphocyte layer is removed. Cells are subjected to homogenization to disrupt cell membranes and the homogenized lymphocytes may be stored (at −80°C) until needed. The antigenic component of such lymphocytic preparations appears to be highly stable, remaining immunologically "intact" at refrigerated temperatures for at least eight months and capable of withstanding at least four "freeze-thaw" cycles (−80° to 22°C).

Example 2

Antibody preparations of the invention may be obtained according to the following illustrative procedure. Six ml of homogenized lymphocytes, prepared according to Example 1 and obtained from one or more patients positively clinically diagnosed for multiple sclerosis, is added to 4 ml of Freund's Complete Adjuvant and mixed to form a stable suspension. Rabbits are each given four 0.5 ml intramuscular injections of the lymphocyte/adjuvant suspension (two on each side) and 1 ml of homogenized lymphocytes without added adjuvant is administered to the marginal ear vein. Intramuscular injections are repeated at 7, 14 and 21 days after the initial inoculation. Two weeks after the last of the injections the animals are sacrificed and the blood is removed by cardiac puncture.

Serum is separated from whole blood and subjected to purification to remove non-specific antibodies and especially those raised in the animal in response to human lymphocytic constituents other than the multiple sclerosis antigenic component. Such separation is accomplished, for example, by serial absorptive contacts with lymphocyte homogenize preparations from non-diseased control patients. Four serial contacts of one hour duration are adequate to "purify" the serum with, e.g., a 4 ml serum sample being treated with 1 ml aliquots of control lymphocyte homogenize at 37°C. Antibody preparations so obtained are quite stable. Preliminary electrophoretic analysis indicates that specific multiple sclerosis antibody is likely to be a microglobulin.

Example 3

Diagnostic testing of patient lymphocyte samples may be effected according to the following exemplary procedure. Employed are commercial immunodiffusion templates (Clinical Sciences, Inc.) having a central well and six additional wells radially outwardly spaced at a uniform distance from the central well. The gel medium is noble agar in 0.1 N barbitol buffer. A well volume of about 0.12 ml is appropriate. About 0.15 ml of antibody preparation (serum) according to Example 2 is placed in the central well. To three of the peripheral wells is added about 0.12 ml of lymphocyte homogenize obtained according to Example 1 from a clinically diagnosed multiple sclerosis patient. To each of the remaining three cells is added about 0.12 ml of lymphocyte homogenize obtained from the test patient. Templates are allowed to incubate at room temperature for 12 to 18 hours and then immersed in 0.85% saline for 4 hours at room temperature. Templates are then visually read for presence or absence of precipitin bands between the central and peripheral wells. Bands appearing between the central well and test sample wells and duplicating those between the central well and multiple sclerosis controls are indicative of a multiple sclerosis disease state in the patient. Absence of bands is read as being indicative of absence of the disease state.

**0 071 654**

Example 4

Operability of the exemplary procedures of Example 3 is evidenced by the following results of two series of tests.

Table 1 below relates the results of 50 diagnostic tests performed on lymphocyte homogenizates obtained from residents of the Erie, Pennsylvania area. As indicated, lymphocytes were obtained from non-multiple sclerosis ("normal") patients, from clinically diagnosed multiple sclerosis patients ("MS"), and from patients clinically diagnosed for a central nervous system disease state other than multiple sclerosis (stroke). Immunodiffusion test data are designated "Pos" for a positive multiple sclerosis result and "Neg" for absence of the indicative precipitin bands. Test/clinical diagnosis correlation is signified by a "+", and a "−" signifies absence of such correlation.

TABLE 1

| Sample number | Test result | Clinical diagnosis | Test/clinical diagnosis correlation |
|---|---|---|---|
| 1 | Pos | MS | + |
| 2 | Neg | Normal | + |
| 3 | Pos | MS | + |
| 4 | Pos | MS | + |
| 5 | Neg | Normal | + |
| 6 | Neg | Normal | + |
| 7 | Pos | MS | + |
| 8 | Neg | Stroke | + |
| 9 | Pos | MS | + |
| 10 | Neg | MS | − |
| 11 | Neg | Normal | + |
| 12 | Pos | MS | + |
| 13 | Neg | Normal | + |
| 14 | Pos | Stroke | −(a) |
| 15 | Neg | MS | − |
| 16 | Pos | MS | + |
| 17 | Pos | MS | + |
| 18 | Pos | MS | + |
| 19 | Pos | MS | + |
| 20 | Neg | Stroke | + |
| 21 | Neg | Normal | + |
| 22 | Pos | MS | + |
| 23 | Pos | Normal | − |
| 24 | Neg | Normal | + |
| 25 | Neg | Normal | + |

4

TABLE 1 (continued)

| Sample number | Test result | Clinical diagnosis | Test/clinical diagnosis correlation |
|---|---|---|---|
| 26 | Neg | Normal | + |
| 27 | Neg | Stroke | + |
| 28 | Neg | Normal | + |
| 29 | Pos | MS | + |
| 30 | Pos | MS | + |
| 31 | Pos | MS | + |
| 32 | Neg | Normal | + |
| 33 | Pos | MS | + |
| 34 | Pos | MS | + |
| 35 | Neg | MS | − |
| 36 | Pos | MS | + |
| 37 | Pos | MS | + |
| 38 | Neg | MS | − |
| 39 | Pos | MS | + |
| 40 | Neg | Normal | + |
| 41 | Neg | MS | − |
| 42 | Neg | Normal | + |
| 43 | Pos | MS | + |
| 44 | Pos | MS | + |
| 45 | Pos | MS | + |
| 46 | Pos | MS | + |
| 47 | Pos | Stroke | − |
| 48 | Neg | Normal | + |
| 49 | Neg | Normal | + |
| 50 | Neg | Normal | + |

(a) Clinical diagnosis later changed to MS.

5

Table 2 below relates to the results of 33 diagnostic tests performed on lymphocyte homogenizates obtained from residents of the Kalamazoo, Michigan area. Once again, samples were obtained from normal, multiple sclerosis and other CNS disease patients as indicated.

TABLE 2

| Sample number | Test result | Clinical diagnosis | Test/clinical diagnosis correlation |
|---|---|---|---|
| 1 | Neg | Normal | + |
| 2 | Neg | Normal | + |
| 3 | Neg | MS | − |
| 4 | Neg | Bell's Palsy | + |
| 5 | Neg | Diabetes | + |
| 6 | Neg | SLE(a) | + |
| 7 | Neg | Stroke | + |
| 8 | Neg | Normal | + |
| 9 | Neg | Normal | +(b) |
| 10 | Neg | Normal | +(c) |
| 11 | Neg | Epilepsy, Sickle Cell | + |
| 12 | Neg | Normal | + |
| 13 | Pos | Normal | −(b) |
| 14 | Pos | SLE | − |
| 15 | Neg | MS | − |
| 16 | Neg | Normal | + |
| 17 | Neg | MS | − |
| 18 | Neg | Stroke | + |
| 19 | Pos | SLE | − |
| 20 | Neg | Stroke | + |
| 21 | Neg | Normal | + |
| 22 | Neg | MS | − |
| 23 | Neg | SLE | + |
| 24 | Neg | Epilepsy | + |
| 25 | Neg | SLE | + |

**0 071 654**

TABLE 2 (continued)

| Sample number | Test result | Clinical diagnosis | Test/clinical diagnosis correlation |
|---|---|---|---|
| 26 | Neg | Normal | + |
| 27 | Neg | Parkinson's | + |
| 28 | Neg | Stroke | + |
| 29 | Neg | Parkinson's | + |
| 30 | Neg | Normal | + |
| 31 | Neg | Normal | + |
| 32 | Neg | Normal | + |
| 33 | Pos | MS | + |

(a) Systemic Lupus Erythematosus
(b) Son of MS patient
(c) Wife of MS patient

Consistent with the foregoing disclosure and without departing from the scope of the appended claims, numerous modifications and variations in practice of the invention are expected to occur to those skilled in that art. As previously noted, antibody preparations of the invention are expected to be useful in providing a variety of immunological diagnostic reagents. In addition to usefulness in immunodiffusion assays, serum containing antibodies may be employed to sensitize immunologically inert particles (stabilized erythrocytes, latex beads and the like) which may be employed in agglutination tests. Isolation and purification techniques well known in the art may be applied to the antibody preparations to secure the active component in more concentrated state which, in turn, may be employed in a 'labeled" form as a reagent in fluorescent antibody and radioimmunoassay diagnostic techniques. Concentration of specific antibodies from serum antibody preparations is also expected to make possible the isolation and characterization of the specific lymphocyte antigen associated with the multiple sclerosis disease state together with antigenic components which currently appear to be present in substantially lesser concentrations in serum of multiple sclerosis patients. The antigen, in purified form, may be usefully employed not only in facilitating large scale quantitative antibody preparation but also as a diagnostic reagent component, especially in immunoassays of a "competitive" type. Such a purified antigen is likely to be useful as a therapeutic agent in the treatment of multiple sclerosis according to therapeutic techniques commonly known as provocative therapy. According to such techniques, low dosages of a disease-causitive antigenic substance are administered for the purpose of provoking a palliative systemic response in the patient.

**Claims**

1. A diagnostic reagent comprising antibodies immunologically reactive with lymphocytic components specifically associated with a multiple sclerosis disease state wherein antibodies are provided as a component of heterologous species serum.

2. A diagnostic method for determining the presence of a multiple sclerosis disease state in a patient, said method comprising the steps of:

(a) forming a mixture of lymphocytic components of a blood sample of said patient with a diagnostic reagent comprising antibodies immunologically reactive with lymphocytic components specifically as associated with a multiple sclerosis disease state; wherein antibodies are provided as a component of heterologous species serum; and

(b) monitoring said mixture for an immunological reaction.

3. A method for preparation of antibodies specifically immunologically reactive with lymphocytic components specifically associated with a multiple sclerosis disease state wherein antibodies are provided as a component of heterologous species serum said method comprising

(a) administering to an immunologically active host animal a preparation of homogenized lymphocytes of a patient having multiple sclerosis; and

(b) isolating antibodies from the blood of the host animal.

4. A method according to claim 3 wherein said isolation step includes contacting serum obtained from

7

said host animal with homogenized lymphocytes of a patient not having multiple sclerosis, whereby antibodies not specific for multiple sclerosis lymphocytic components are removed from the serum.

5. An antigenic material, suitable for use as an inoculum to generate specific multiple sclerosis antibodies wherein antibodies are provided as a component of heterologous species serum, said antigenic material comprising an homogenizate of lymphocytes of a patient having multiple sclerosis.

## Patentansprüche

1. Diagnostisches Reagenz, welches immunologisch reaktive Antikörper mit lymphozytischen Bestandteilen enthält, die mit dem Krankheitszustand der multiplen Sklerose in spezifischem Zusammenhang stehen, wobei die Antikörper als Bestandteil des Blutserums einer heterologen Spezies bereitgestellt werden.

2. Diagnostisches Verfahren zum Bestimmen des Vorhandenseins eines Krankheitszustandes der multiplen Sklerose in einem Patienten, wobei das besagte Verfahren die Schritte:

(a) Herstellen einer Mischung von lymphozytischen Bestandteilen einer Blutprobe des besagten Patienten mit einem diagnostischen Reagenz, welches immunologisch reaktive Antikörper mit lymphozytischen Bestandteilen enthält, die mit dem Krankheitszustand der multiplen Sklerose in spezifischem Zusammenhang stehen, wobei die Antikörper als Bestandteile des Blutserums einer heterologen Spezies bereitgestellt werden; und

(b) Überwachen besagter Mischung auf eine immunologische Reaktion enthält.

3. Verfahren zur Gewinnung von spezifisch immunologisch reaktiven Antikörpern mit lymphozytischen Bestandteilen, die mit dem Krankheitszustand der multiplen Sklerose in spezifischem Zusammenhang stehen, wobei die Antikörper als Bestandteil des Blutserums einer heterologen Spezies bereitgestellt werden; wobei besagtes Verfahren

(a) Verabreichen einer Zubereitung von homogenisierten Lymphozyten eines Patienten mit multipler Sklerose an ein immunologisch aktives Wirtstier; und

(b) Isolieren der Antikörper aus dem Blut des Wirtstieres enthält.

4. Verfahren nach Anspruch 3, wobei der besagte Isolationsschritt das Zusammenbringen des Serums, das von besagtem Writstier erhalten wurde, mit homogenisierten Lymphozyten eines Patienten ohne multiple Sklerose einschließt, wodurch diejenigen Antikörper, die nicht spezifisch für die lymphozytischen Bestandteile der multiplen Sklerose sind, aus dem Serum entfernt werden.

5. Antigenmaterial, welches zur Verwendung als Impfmaterial geeignet ist, um spezifische Antikörper gegen multiple Sklerose zu erzeugen, wobei die Antikörper als Bestandteil des Blutserums einer heterologen Spezies bereitgestellt werden; wobei besagtes Antigenmaterial ein Homogenisat von Lymphozyten eines Patienten mit multipler Sklerose enthält.

## Revendications

1. Réactif de diagnostic comprenant des anticorps immunologiquement réactifs avec des composants lymphocytiques spécifiquement associés à un état morbide de sclérose en plaques, dans lequel les anticorps sont fournis comme composant de sérum d'espèces hétérologues.

2. Procédé de diagnostic pour déterminer la présence d'un état morbide de sclérose en plaques chez un patient, ledit procédé comprenant les étapes de:

a) créer un mélange de composants lymphocytiques d'un échantillon sanguin dudit patient avec un réactif de diagnostic comprenant des anticorps immunologiquement réactifs avec les composants lymphocytiques spécifiquement associés à un état morbide de sclérose en plaques, lesdits anticorps étant fournis comme composant de sérum d'espèces hétérologues; et

b) contrôler ledit mélange pour une réaction immunologique.

3. Procédé de préparation d'anticorps spécifiquement immunologiquement réactifs avec des composants lymphocytiques spécifiquement associés à un état morbide de sclérose en plaques, lesdits anticorps étant fournis comme composant de sérum d'espèces hétérologues, ledit procédé comprenant:

a) l'administration à un animal hôte immunologiquement actif d'une préparation de lymphocytes homogénéisés d'un patient ayant la sclérose en plaques; et

b) l'isolation des anticorps du sang de l'animal hôte.

4. Procédé selon la revendication 3, caractérisé en ca que ladite étape d'isolation comprend la mise en contact du sérum obtenu à partir dudit animal hôte avec des lymphocytes homogénéisés d'un patient n'ayant pas la sclérose en plaques, par quoi les anticorps non spécifiques des composants lymphocytiques de la sclérose en plaques sont retirés du sérum.

5. Matériau antigènique, adapté pour être utilisé comme agent d'inoculation pour créer des anticorps spécifiques de la sclérose en plaques, lesdits anticorps étant fournis comme composant de sérum d'espèces hétérologues, ledit matériau antigènique comprenant un homogénéisat de lymphocytes d'un patient ayant la sclérose en plaques.